(19) Europäisches Patentamt
European Patent Office
Office européen des brevets

(11) **EP 4 708 261 A1**

(12) **EUROPEAN PATENT APPLICATION**
published in accordance with Art. 153(4) EPC

(43) Date of publication:
**11.03.2026 Bulletin 2026/11**

(21) Application number: **24800026.7**

(22) Date of filing: **04.03.2024**

(51) International Patent Classification (IPC):
**G09C 1/00** *(2006.01)*

(52) Cooperative Patent Classification (CPC):
**G06F 21/71; G09C 1/00**

(86) International application number:
**PCT/JP2024/008140**

(87) International publication number:
**WO 2024/228295 (07.11.2024 Gazette 2024/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.05.2023 JP 2023075875**

(71) Applicant: **NTT DOCOMO BUSINESS, Inc.
Tokyo 100-8019 (JP)**

(72) Inventors:
• **TANAKA, Satoshi**
  **Tokyo 100-8019 (JP)**
• **SAKURAI, Yoichi**
  **Tokyo 100-8019 (JP)**
• **SAWADA, Masashi**
  **Tokyo 100-8019 (JP)**

(74) Representative: **Hoffmann Eitle
Patent- und Rechtsanwälte PartmbB
Arabellastraße 30
81925 München (DE)**

(54) **ANALYSIS DEVICE, ANALYSIS METHOD, AND ANALYSIS PROGRAM**

(57)     An analysis device according to the embodiment includes a test unit and an output control unit. The test unit performs a test of Cox proportional hazard regression analysis by secure computation by using a partial regression coefficient, a Hessian matrix, or a score vector, or any combination thereof obtained by the Cox proportional hazard regression analysis by secure computation. The output control unit outputs a result of the test by the test unit.

FIG.2

EP 4 708 261 A1

**Description**

[Technical Field]

The present invention relates to an analysis device, an analysis method, and an analysis program.

[Background Art]

In the related art, a secure computation system that performs statistical calculation while keeping data secret and provides a user with a statistic obtained as a result of the calculation is known. For example, the secure computation system may be used for analysis of data in a medical field or the like that handles important personal information.

In addition, a secure computation system that performs statistical processing on data in an encrypted state is known. For example, a technique for obtaining a parameter of logistic regression analysis using the data in an encrypted state is known (See, for example, Patent Literature 2).

In addition, Cox proportional hazard regression analysis that is a method similar to logistic regression analysis and is used for survival time analysis is known (See, for example, Patent Literature 3).

[Citation List]

[Patent Citation]

Patent Literature 1: International Publication Pamphlet No. WO 2019/124260 A
Patent Literature 2: Japanese Laid-open Patent Publication No. 2020-042128 A
Patent Literature 3: Japanese Laid-open Patent Publication No. 2008-299370 A

[Non Patent Citation]

Non Patent Literature 1: NTT Corp., System of Secure Computation and Principles thereof, (online), (searched on January 6, 2023), Internet <URL:https://www.rd.ntt/sil/project/sc/secure_computation.h tml>

[Summary of Invention]

[Technical Problem]

However, the technique in the related art has a problem that it may be difficult to perform a test of Cox proportional hazard regression analysis performed by secure computation.

[Solution to Problem]

In order to solve the above-described problems and achieve the object, an analysis device according to the present invention includes: a test unit that performs a test of Cox proportional hazard regression analysis by secure computation by using a partial regression coefficient, a Hessian matrix, or a score vector, or any combination thereof obtained by the Cox proportional hazard regression analysis by secure computation; and an output control unit that outputs a result of the test by the test unit.

[Advantageous Effects of Invention]

According to the present invention, a test of Cox proportional hazard regression analysis performed by secure computation can be performed.

[Brief Description of Drawings]

FIG. 1 is a diagram illustrating a configuration example of an analysis system according to an embodiment.
FIG. 2 is a diagram illustrating a configuration example of an analysis device according to the embodiment.

FIG. 3 is a diagram illustrating an example of learning data.

FIG. 4 is a diagram illustrating definitions of symbols.

FIG. 5 is a flowchart illustrating a flow of a Wald test.

FIG. 6 is a flowchart illustrating a flow of a Score test.

FIG. 7 is a flowchart illustrating a flow of a likelihood ratio test.

FIG. 8 is a flowchart illustrating a flow of processing of calculating log partial likelihood.

FIG. 9 is a diagram illustrating an example of a computer that executes an analysis program.

[Embodiments for Carrying Out the Invention]

Hereinafter, embodiments of an analysis device, an analysis method, and an analysis program according to the present application are described in detail with reference to the drawings. Note that the present invention is not limited to the embodiments described below.

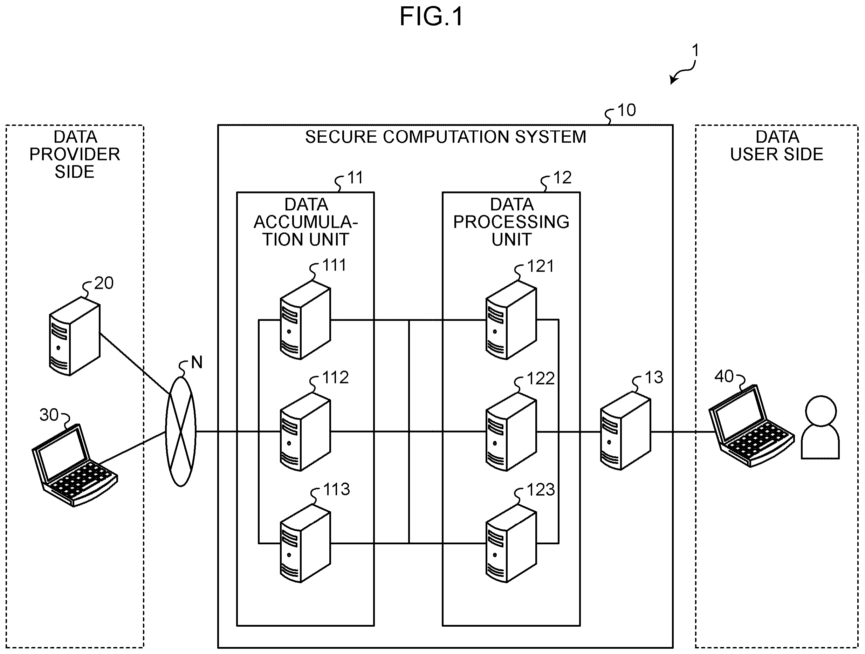

First, a configuration of an analysis system is described with reference to FIG. 1. The analysis system is a system for analyzing data using secure computation.

As illustrated in FIG. 1, an analysis system 1 includes a secure computation system 10. Furthermore, the secure computation system 10 is connected to a providing device 20 and a providing device 30 via a network N. For example, the network N is the Internet. In addition, the secure computation system 10 is connected to a terminal device 40.

The providing device 20 and the providing device 30 are devices on the data provider side. The providing device 20 and the providing device 30 provide (register) data to the secure computation system 10.

The data provided by the providing device 20 and the providing device 30 includes information (for example, personal information such as a name and an address of an individual) which is desirably concealed. For example, the providing device 20 and the providing device 30 provide medical care data or health examination data used in a medical institution. However, the data provided by the providing device 20 and the providing device 30 is not limited to data used in a medical institution.

The secure computation system 10 includes a data accumulation unit 11 and a data processing unit 12. The data accumulation unit 11 includes a plurality of accumulation devices (an accumulation device 111, an accumulation device 112, and an accumulation device 113) that accumulate data by secret sharing. In addition, the data processing unit 12 includes a plurality of calculation devices (a calculation device 121, a calculation device 122, and a calculation device 123) that process data by secure computation. Note that the number of accumulation devices and the number of calculation devices are not limited to the example illustrated in FIG. 1.

The secure computation system 10 can perform secret sharing and secure computation according to the method described in Non-Patent Literature 1 (posted URL: https://www.rd.ntt/sil/project/sc/secure_computation.html).

First, the data provided to the secure computation system 10 is divided (fragmented) into a plurality of shares. Then, the plurality of shares are distributed into and accumulated in a plurality of accumulation devices included in the data accumulation unit 11. In the example of FIG. 1, the provided data is divided into three shares. Then, the accumulation device 111, the accumulation device 112, and the accumulation device 113 accumulate shares one by one.

The data processing unit 12 performs secure computation on the share accumulated in the data accumulation unit 11. The data processing unit 12 executes secure computation by multi-party computation using a plurality of calculation devices. In the example of FIG. 1, the data processing unit 12 executes secure computation by the calculation device 121, the calculation device 122, and the calculation device 123.

The data processing unit 12 can perform various statistical operations without restoring the share. For example, the data processing unit 12 can perform an operation of a table such as sorting and combining, aggregation of the number of records, calculation of statistics such as a total sum, an average, a maximum value, a minimum value, and a sample variance, and a statistical test such as t-test. Furthermore, the data processing unit 12 can perform statistical analysis such as regression analysis and principal component analysis.

An analysis device 13 analyzes data using the data processing unit 12. The analysis device 13 provides an analysis result to the terminal device 40 on the data user side based on the result of the secure computation executed by the data processing unit 12. The user can obtain an analysis result of data via the terminal device 40.

For example, the secure computation system 10 may be provided with data related to attributes and bodies for each individual. The data related to the attribute and the body is personal information that is desirably concealed. The data related to the attributes and the bodies includes, for example, ages, genders, heights, weights, and the like. The data accumulation unit 11 stores a share obtained by fragmenting the provided data in each accumulation device.

Note that each divided share is data that is singly meaningless. Therefore, the original data cannot be restored from one share. Meanwhile, it is possible to restore the original data by gathering a plurality of shares.

The user of the data cannot view the registered data itself but can view the analysis result of the data via the analysis device 13 and the terminal device 40. For example, when the data includes the gender and the weight of an individual, the user cannot view the gender and the weight of each individual but can view the "average weight of men" that is an analysis result of the data.

As an example, the data accumulation unit 11 can perform secret sharing by using a technique referred to as Shamir's threshold secret sharing method. At this time, the data accumulation unit 11 stores, as shares, three coordinates passing through a polynomial having the original data as an intercept in each server. In addition, since the inclination of the polynomial is randomly determined, even if the original data is the same, the share is not necessarily the same every time. The original data may be a numerical value or data converted into a numerical value.

The secure computation system 10 can restore the original data from a plurality of shares. If the polynomial is a linear expression, the secure computation system 10 can obtain the intercept (corresponding to the original data) from the intersection of a straight line connecting the two coordinates (corresponding to the share) and an axis. Meanwhile, since a straight line is not determined from one coordinate, the original data cannot be restored.

In addition, as described above, the data processing unit 12 can perform secure computation on the original data without restoring the share. For example, the result of adding the shares represented by the coordinates corresponds to the share of the result of adding the original data of each share.

The analysis device 13 causes the data processing unit 12 to execute processing by secure computation in response to a request from the terminal device 40. Note that the data processing unit 12 or the terminal device 40 may embody a function equivalent to that of the analysis device 13. For example, the analysis system 1 may be a configuration not including the analysis device 13. In that case, the terminal device 40 is connected to the data processing unit 12 and executes processing equivalent to that of the analysis device 13. Furthermore, the statistical operation based on the share may be executed by the terminal device 40 instead of the data processing unit 12.

In the first embodiment, an example is described in which the analysis device 13 performs Cox proportional hazard regression analysis and a test of the Cox proportional hazard regression analysis by secure computation.

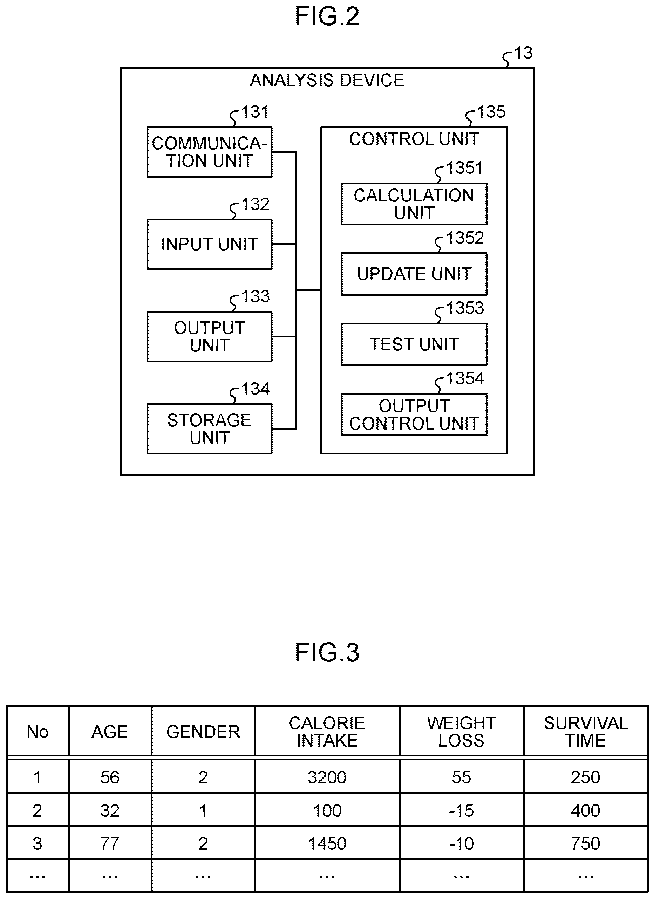

A configuration of the analysis device 13 is described with reference to FIG. 2. FIG. 2 is a diagram illustrating a configuration example of the analysis device according to the embodiment.

Each unit of the analysis device 13 is described. As illustrated in FIG. 2, the analysis device 13 includes a communication unit 131, an input unit 132, an output unit 133, a storage unit 134, and a control unit 135.

The communication unit 131 performs data communication between other devices. For example, the communication unit 131 is a network interface card (NIC). The communication unit 131 can transmit and receive data to and from other devices.

The input unit 132 is an interface for receiving input of data. The input unit 132 is connected, for example, to an input device such as a mouse and a keyboard.

The output unit 133 is an interface for outputting data. The output unit 133 is connected, for example, to an input device such as a display and a speaker.

The storage unit 134 is a storage device such as a hard disk drive (HDD), a solid state drive (SSD), or an optical disk. Note that the storage unit 134 may be a semiconductor memory capable of rewriting data, such as a random access memory (RAM), a flash memory, or a non volatile static random access memory (NVSRAM). The storage unit 134 stores an operating system (OS) and various programs executed by the analysis device 13.

The control unit 135 controls the entire analysis device 13. The control unit 135 is, for example, an electronic circuit such as a central processing unit (CPU), a micro processing unit (MPU), or a graphics processing unit (GPU), or an integrated circuit such as an application specific integrated circuit (ASIC) or a field programmable gate array (FPGA). In addition, the control unit 135 includes an internal memory for storing programs and control data defining various processing procedures and executes each process using the internal memory.

The control unit 135 functions as various processing units by various programs operating. For example, the control unit 135 includes a calculation unit 1351, an update unit 1352, a test unit 1353, and an output control unit 1354.

The calculation unit 1351 calculates Cox proportional hazard regression by secure computation. The calculation unit 1351 inputs an explanatory variable to a Cox proportional hazard regression model and outputs an objective variable.

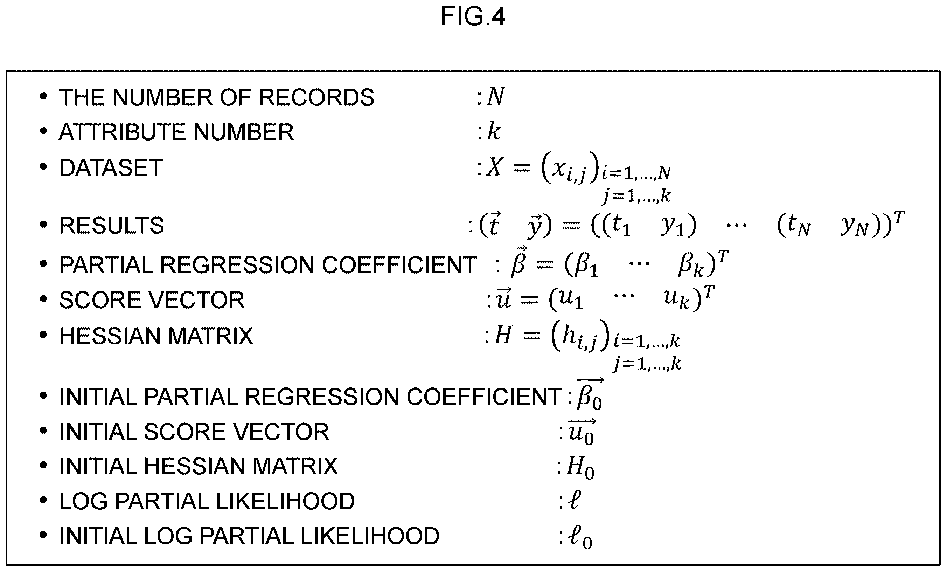

FIG. 4 illustrates definitions of symbols used in the following description. FIG. 4 is a diagram illustrating definitions of the symbols.

As illustrated in FIG. 4, N is the number of records. k is the number of attributes (explanatory variables). A dataset X includes attribute information of each record of the N×k matrix. For example, the i-th record is $(x_1,..., x_k)$.

The result is information indicating survival or death of a certain record. t represents observation time. y is a binary value of 0 (survival) or 1 (death).

A partial regression coefficient and a score vector are represented by column vectors. The score vector is a first derivative of the log partial likelihood. Also, the Hessian matrix is a second derivative of the log partial likelihood.

In addition, a subscript 0 is attached to a partial regression coefficient, a score vector, a Hessian matrix, and a log partial likelihood. A method of calculating the log partial likelihood is described below.

The update unit 1352 updates a parameter of the Cox proportional hazard regression model by secure computation so that the objective variable calculated by the calculation unit 1351 approaches a correct value.

The Cox proportional hazard regression model is learned by performing the processing of the calculation unit 1351 and the update unit 1352 once or a plurality of times.

FIG. 3 is a diagram illustrating an example of learning data. Values in an "age" column, a "gender" column, a "calorie intake" column, and a "weight loss" column in FIG. 3 are explanatory variables of the Cox proportional hazard regression model. Furthermore, a predicted survival rate based on the value of the "survival time" column is an objective variable of the Cox proportional hazard regression model and corresponds to the hazard function h (t|x).

The calculation unit 1351 inputs the values in the "age" column, the "gender" column, the "calorie intake" column, and the "weight loss" column to the Cox proportional hazard regression model and obtains an output. The update unit 1352 updates the parameter of the Cox proportional hazard regression model so that the output obtained by the calculation unit 1351 approaches the predicted survival rate based on the value of the "survival time" column.

The test unit 1353 performs the test of the Cox proportional hazard regression model. In the secure computation, it is difficult to acquire the learning data itself from the outside. Therefore, the test unit 1353 calculates a statistic for the test based on limited information that can be acquired.

Here, the test unit 1353 performs the test of the Cox proportional hazard regression analysis by secure computation using the partial regression coefficient, the Hessian matrix, or the score vector, or any combination thereof obtained by the Cox proportional hazard regression analysis by secure computation. The test unit 1353 acquires information related to the Cox proportional hazard regression analysis such as the partial regression coefficient, the Hessian matrix, and the score vector from the secure computation system 10.

Processing when the test unit 1353 performs the Wald test, the Score test, and the likelihood ratio test is described.

(Wald Test)

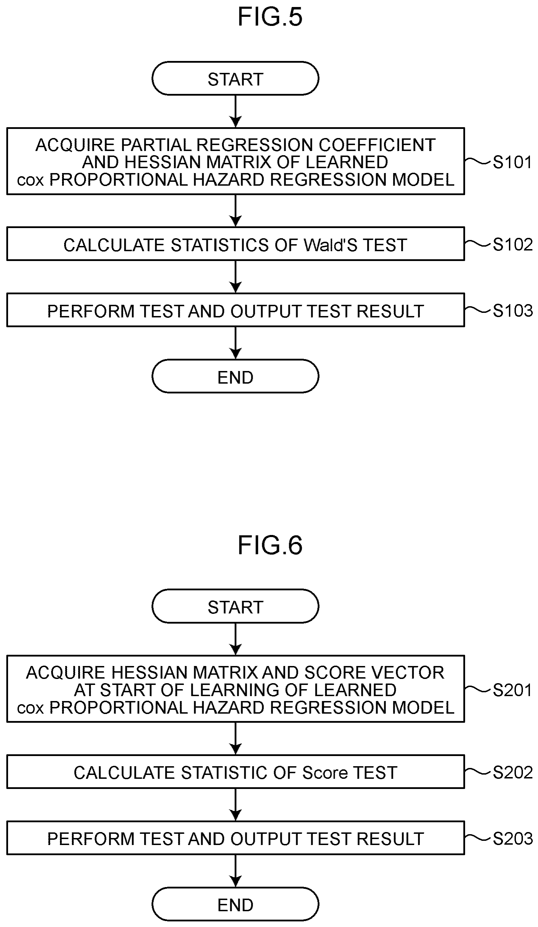

FIG. 5 is a flowchart illustrating a flow of the Wald test. As illustrated in FIG. 5, the test unit 1353 acquires the partial regression coefficient and the Hessian matrix of the learned Cox proportional hazard regression model from the secure computation system 10 (Step S101).

Next, the test unit 1353 calculates the statistic of the Wald test as in Formula (1) (Step S102). Then, the test unit 1353 executes the test and outputs the test result via the output control unit 1354 (Step S103). However, when an initial partial regression coefficient is set to 0, the test unit 1353 calculates the statistic by Formula (2).

$$\left(\vec{\beta} - \vec{\beta_0}\right)^T H \left(\vec{\beta} - \vec{\beta_0}\right) \qquad \cdots (1)$$

$$\vec{\beta}^T H \vec{\beta}$$
$$\left(\text{When } \vec{\beta_0} = \vec{0}\right) \qquad \cdots (2)$$

In this manner, the test unit 1353 calculates the statistic of the Wald test based on the partial regression coefficient and the Hessian matrix and performs the $\chi$-square test based on the statistic.

(Score Test)

FIG. 6 is a flowchart illustrating a flow of the Score test. As illustrated in FIG. 6, the test unit 1353 acquires the Hessian matrix (initial Hessian matrix in FIG. 4) and the score vector (initial score vector in FIG. 4) at the start of learning of the learned Cox proportional hazard regression model from the secure computation system 10 (Step S201).

Next, the test unit 1353 calculates the statistic of the Score test as in Formula (3) (Step S202). Then, the test unit 1353 executes the test and outputs the test result via the output control unit 1354 (Step S203).

$$\vec{u_0}^t H_0 \vec{u_0} \qquad \cdots (3)$$

As described above, the test unit 1353 calculates the statistic of the Score test based on the Hessian matrix and the score vector at the start of learning of the regression model of the Cox proportional hazard regression analysis and performs the $\chi$-square test based on the statistic.

(Likelihood Ratio Test)

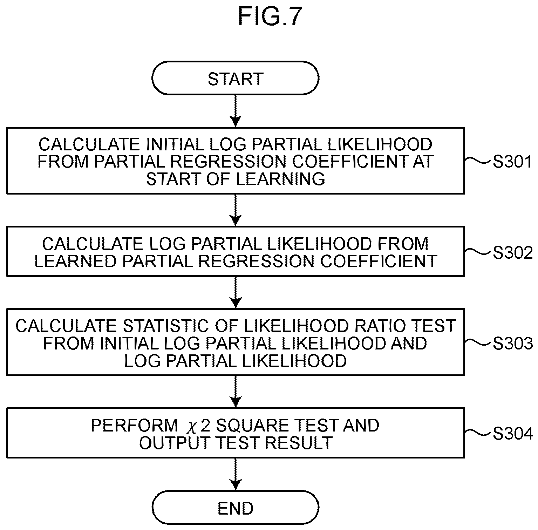

FIG. 7 is a flowchart illustrating a flow of the likelihood ratio test. The test unit 1353 acquires the partial regression coefficient and the Hessian matrix of the learned Cox proportional hazard regression model from the secure computation system 10.

Furthermore, each record of the learning data of FIG. 3 corresponds to each patient, and the hazard function of the Cox proportional hazard regression model represents the survival probability of the patient at a certain time. From the survival time of the learning data, it is derived whether the patient has died at a certain time.

Among the records of the learning data, a record corresponding to a patient who dies at a designated time is referred to as a death record. Also, among the records of the learning data, a record corresponding to a patient who does not die at a designated time is referred to as a remaining record.

As illustrated in FIG. 7, first, the test unit 1353 calculates an initial log partial likelihood from the partial regression coefficient at the start of learning (Step S301). In addition, the test unit 1353 calculates a log partial likelihood from the learned partial regression coefficient (Step S302).

The test unit 1353 calculates the log partial likelihood by Formula (4). In addition, the test unit 1353 calculates the initial log partial likelihood by a formula in which $\beta$ in Formula (4) is replaced with $\beta_0$. Details of the calculation of the log partial likelihood are described below.

$$\ell = \sum_{i=1}^{N} \left\{ y_i \left( \vec{x_t} \cdot \vec{\beta} - \log \left( \sum_{j=i}^{N} \exp(\vec{x_t} \cdot \vec{\beta}) \right) \right) \right\} \qquad \cdots (4)$$

Subsequently, the test unit 1353 calculates the statistic of the likelihood ratio test from the initial log partial likelihood and the log partial likelihood as in Formula (5) (Step S303). Then, the test unit 1353 performs the $\chi$-square test and outputs a test result (Step S304).

$$2(\ell - \ell_0) \qquad \cdots (5)$$

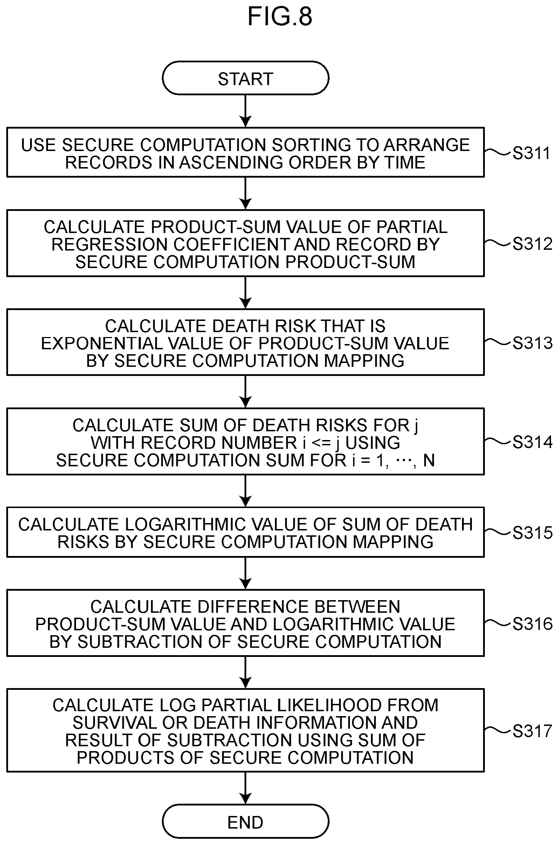

FIG. 8 is a flowchart illustrating a flow of processing of calculating the log partial likelihood. As illustrated in FIG. 8, first, the test unit 1353 uses secure computation sorting to arrange the records of the dataset in ascending order by time (Step S311).

Next, the test unit 1353 calculates a product-sum value of the partial regression coefficient and the record by secure computation product-sum (Step S312). Furthermore, the test unit 1353 calculates a death risk (exp on the right side of the Formula (4)) that is the exponential value of the product-sum value, by the secure computation mapping (Step S313).

Here, for $i = 1, ..., N$, the test unit 1353 calculates the sum of the death risks for j with the record number $i <= j$ (right $\Sigma$ on the right side of Formula (4)) using the secure computation sum (Step S314).

Subsequently, the test unit 1353 calculates a logarithmic value of the sum of the death risks (log on the right side of Formula (4)) by the secure computation mapping (Step S315).

Then, the test unit 1353 calculates the difference between the product-sum value and the logarithmic value by subtraction of secure computation (subtraction on the right side of Formula (4)) (Step S316). Furthermore, the test unit 1353 calculates the log partial likelihood from the survival or death information ($y_i$ in Formula (4)) and the result of the subtraction using the sum of products of the secure computation (Step S317).

As described above, the test unit 1353 the test unit 1353 calculates, as a first death risk, the sum of products of the death record and the partial regression coefficient used for learning of the regression model of the Cox proportional hazard regression analysis by secure computation, calculates, as a second death risk, the sum of products of the remaining record and the partial regression coefficient used for learning of the regression model by secure computation, calculates the log likelihood based on the first death risk and the second death risk, and performs a test based on the log likelihood.

(Effects of Embodiment)

As described above, the analysis device 13 includes the test unit 1353 and the output control unit 1354. The test unit 1353 performs the test of the Cox proportional hazard regression analysis by secure computation using the partial regression coefficient, the Hessian matrix, or the score vector, or any combination thereof obtained by the Cox proportional hazard regression analysis by secure computation. The output control unit 1354 outputs the result of the test by the test unit 1353.

As a result, the analysis device 13 can perform a test of Cox proportional hazard regression analysis performed by secure computation.

For example, the test unit 1353 calculates the statistic of the Wald test based on the partial regression coefficient and the Hessian matrix and performs the test based on the statistic.

For example, the test unit 1353 calculates the statistic of the Score test based on the Hessian matrix and the score vector at the start of learning of the regression model of the Cox proportional hazard regression analysis and performs the test based on the statistic.

For example, the test unit 1353 calculates, as a first death risk, the sum of products of the death record and the partial regression coefficient used for learning of the regression model of the Cox proportional hazard regression analysis by secure computation, calculates, as a second death risk, the sum of products of the remaining record and the partial regression coefficient used for learning of the regression model by secure computation, calculates the log likelihood based on the first death risk and the second death risk, and performs a test based on the log likelihood.

In this manner, the analysis device 13 can perform the Wald test, the Score test, and the likelihood ratio test.

(System Configuration and the Like)

In addition, each component of each illustrated device is functionally conceptual and does not necessarily need to be physically configured as illustrated. That is, a specific form of distribution and integration of each device is not limited to the illustrated form and can be configured by functionally or physically distributing or integrating all or a part thereof in any unit according to various loads, usage conditions, and the like. Furthermore, all or any part of each processing function performed in each device can be embodied by a central processing unit (CPU) and a program analyzed and executed by the CPU or can be embodied as hardware by wired logic. Note that the program may be executed not only by the CPU but also by another processor such as a GPU.

In addition, among the processes described in the present embodiment, all or some of the processes described as being automatically performed can be manually performed, or all or some of the processes described as being manually performed can be automatically performed by a known method. In addition, the processing procedure, the control procedure, the specific name, and the information including various pieces of data and various parameters illustrated in the document and the drawings can be arbitrarily changed unless otherwise specified.

(Program)

As an embodiment, the analysis device 13 can be implemented by installing an analysis program for executing the above analysis processing as package software or online software in a desired computer. For example, by causing the information processing apparatus to execute the above analysis program, the information processing apparatus can be caused to function as the analysis device 13. The information processing apparatus described here includes a desktop or notebook personal computer. In addition, the information processing apparatus includes mobile communication terminals such as a smartphone, a mobile phone, and a personal handyphone system (PHS), and a slate terminal such as a personal digital assistant (PDA) and the like are included in the category thereof.

Furthermore, the analysis device 13 can also be implemented as an analysis server device that uses, as a client, a terminal device used by the user and provides the client with a service related to the analysis processing. For example, the analysis server device is implemented as a server device that provides an analysis service in which the partial regression coefficient, the Hessian matrix, and the score vector obtained by Cox proportional hazard regression analysis by secure computation are used as inputs, and a test result of the Cox proportional hazard regression analysis is used as an output.

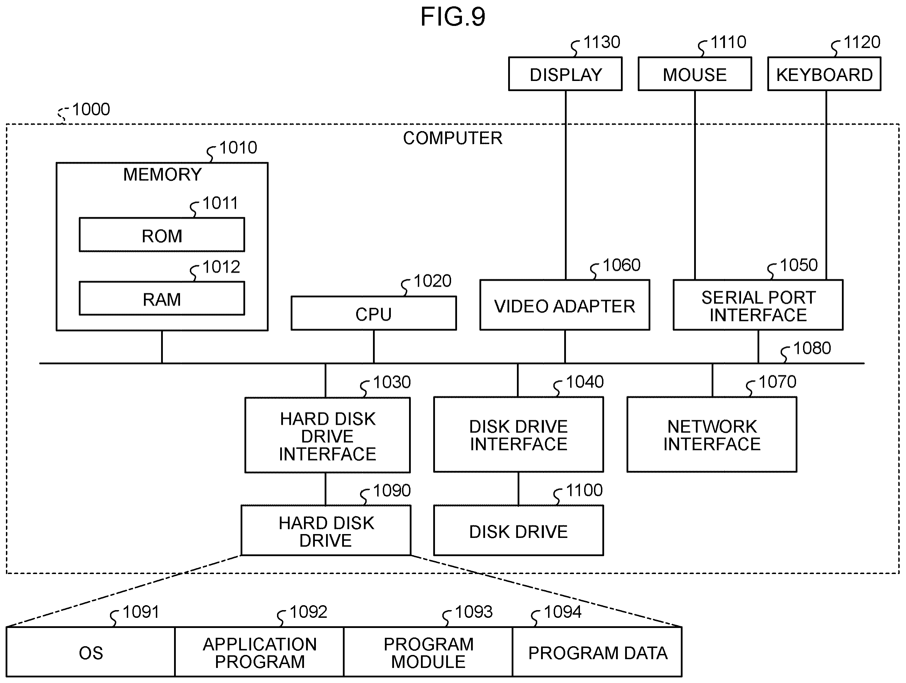

FIG. 9 is a diagram illustrating an example of a computer that executes the analysis program. A computer 1000 includes, for example, a memory 1010 and a CPU 1020. Also, the computer 1000 also includes a hard disk drive interface 1030, a disk drive interface 1040, a serial port interface 1050, a video adapter 1060, and a network interface 1070. These units are connected by a bus 1080.

The memory 1010 includes a read only memory (ROM) 1011 and a random access memory (RAM) 1012. The ROM 1011 stores, for example, a boot program such as a basic input output system (BIOS). The hard disk drive interface 1030 is connected to a hard disk drive 1090. The disk drive interface 1040 is connected to a disk drive 1100. For example, a removable storage medium such as a magnetic disk or an optical disk is inserted into the disk drive 1100. The serial port interface 1050 is connected to, for example, a mouse 1110 and a keyboard 1120. The video adapter 1060 is connected to, for example, a display 1130.

The hard disk drive 1090 stores, for example, an OS 1091, an application program 1092, a program module 1093, and program data 1094. That is, the program that defines each processing of the analysis device 13 is implemented as the program module 1093 in which a code executable by a computer is described. The program module 1093 is stored in, for example, the hard disk drive 1090. For example, the program module 1093 for executing processing similar to the functional configuration in the analysis device 13 is stored in the hard disk drive 1090. Note that the hard disk drive 1090 may be replaced with a solid state drive (SSD).

In addition, the setting data used in the processing of the embodiment described above is stored, for example, in the memory 1010 or the hard disk drive 1090 as the program data 1094. Then, the CPU 1020 reads the program module 1093 and the program data 1094 stored in the memory 1010 and the hard disk drive 1090 to the RAM 1012 as necessary and executes the processing of the embodiment described above.

Note that the program module 1093 and the program data 1094 are not limited to a case of being stored in the hard disk drive 1090 and may be stored in, for example, a detachable storage medium and read by the CPU 1020 via the disk drive 1100 or the like. Alternatively, the program module 1093 and the program data 1094 may be stored in another computer connected via a network (local area network (LAN), wide area network (WAN), and the like). Then, the program module 1093 and the program data 1094 may be read by the CPU 1020 from another computer via the network interface 1070.

[Explanation of Reference]

1 ANALYSIS SYSTEM
10 SECURE COMPUTATION SYSTEM
11 DATA ACCUMULATION UNIT
12 DATA PROCESSING UNIT
13 ANALYSIS DEVICE
131 COMMUNICATION UNIT
132 INPUT UNIT
133 OUTPUT UNIT
134 STORAGE UNIT
135 CONTROL UNIT
1351 CALCULATION UNIT
1352 UPDATE UNIT
1353 TEST UNIT
1354 OUTPUT CONTROL UNIT

**Claims**

1. An analysis device comprising:

   a test unit that performs a test of Cox proportional hazard regression analysis by secure computation by using a partial regression coefficient, a Hessian matrix, or a score vector, or any combination thereof obtained by the Cox proportional hazard regression analysis by secure computation; and
   an output control unit that outputs a result of the test by the test unit.

2. The analysis device according to claim 1, wherein the test unit calculates a statistic of a Wald test based on the partial regression coefficient and the Hessian matrix and performs a test based on the statistic.

3. The analysis device according to claim 1, wherein the test unit calculates a statistic of a Score test based on the Hessian matrix and the score vector at a start of learning of a regression model of the Cox proportional hazard regression analysis and performs a test based on the statistic.

4. The analysis device according to claim 1, wherein the test unit calculates, as a first death risk, a sum of products of a death record and a partial regression coefficient used for learning of a regression model of the Cox proportional hazard regression analysis by secure computation, calculates, as a second death risk, a sum of products of a remaining record and a partial regression coefficient used for learning of the regression model by secure computation, calculates log likelihood based on the first death risk and the second death risk, and performs a test based on the log likelihood.

5. An analysis method executed by an analysis device, the analysis method comprising:

   a test step of performing a test of Cox proportional hazard regression analysis by secure computation by using a partial regression coefficient, a Hessian matrix, or a score vector, or any combination thereof obtained by the Cox proportional hazard regression analysis by secure computation; and
   an output control step of outputting a result of the test at the test step.

**6.** An analysis program that causes a computer to execute a process comprising:

a test step of performing a test of Cox proportional hazard regression analysis by secure computation by using a partial regression coefficient, a Hessian matrix, or a score vector, or any combination thereof obtained by the Cox proportional hazard regression analysis by secure computation; and
an output control step of outputting a result of the test at the test step.

FIG.1

# FIG.2

# FIG.3

| No | AGE | GENDER | CALORIE INTAKE | WEIGHT LOSS | SURVIVAL TIME |
|---|---|---|---|---|---|
| 1 | 56 | 2 | 3200 | 55 | 250 |
| 2 | 32 | 1 | 100 | -15 | 400 |
| 3 | 77 | 2 | 1450 | -10 | 750 |
| ... | ... | ... | ... | ... | ... |

# FIG.4

- THE NUMBER OF RECORDS $: N$
- ATTRIBUTE NUMBER $: k$
- DATASET $: X = \left(x_{i,j}\right)_{\substack{i=1,\ldots,N \\ j=1,\ldots,k}}$
- RESULTS $: (\vec{t} \quad \vec{y}) = ((t_1 \quad y_1) \quad \cdots \quad (t_N \quad y_N))^T$
- PARTIAL REGRESSION COEFFICIENT $: \vec{\beta} = (\beta_1 \quad \cdots \quad \beta_k)^T$
- SCORE VECTOR $: \vec{u} = (u_1 \quad \cdots \quad u_k)^T$
- HESSIAN MATRIX $: H = \left(h_{i,j}\right)_{\substack{i=1,\ldots,k \\ j=1,\ldots,k}}$
- INITIAL PARTIAL REGRESSION COEFFICIENT $: \overrightarrow{\beta_0}$
- INITIAL SCORE VECTOR $: \overrightarrow{u_0}$
- INITIAL HESSIAN MATRIX $: H_0$
- LOG PARTIAL LIKELIHOOD $: \ell$
- INITIAL LOG PARTIAL LIKELIHOOD $: \ell_0$

EP 4 708 261 A1

# FIG.5

```
        START

ACQUIRE PARTIAL REGRESSION COEFFICIENT
    AND HESSIAN MATRIX OF LEARNED             S101
cox PROPORTIONAL HAZARD REGRESSION MODEL

CALCULATE STATISTICS OF Wald'S TEST           S102

PERFORM TEST AND OUTPUT TEST RESULT           S103

        END
```

# FIG.6

```
        START

ACQUIRE HESSIAN MATRIX AND SCORE VECTOR
    AT START OF LEARNING OF LEARNED            S201
cox PROPORTIONAL HAZARD REGRESSION MODEL

CALCULATE STATISTIC OF Score TEST             S202

PERFORM TEST AND OUTPUT TEST RESULT           S203

        END
```

# FIG.7

START

CALCULATE INITIAL LOG PARTIAL LIKELIHOOD FROM PARTIAL REGRESSION COEFFICIENT AT START OF LEARNING — S301

CALCULATE LOG PARTIAL LIKELIHOOD FROM LEARNED PARTIAL REGRESSION COEFFICIENT — S302

CALCULATE STATISTIC OF LIKELIHOOD RATIO TEST FROM INITIAL LOG PARTIAL LIKELIHOOD AND LOG PARTIAL LIKELIHOOD — S303

PERFORM $\chi 2$ SQUARE TEST AND OUTPUT TEST RESULT — S304

END

# FIG.8

START

USE SECURE COMPUTATION SORTING TO ARRANGE RECORDS IN ASCENDING ORDER BY TIME ～S311

CALCULATE PRODUCT-SUM VALUE OF PARTIAL REGRESSION COEFFICIENT AND RECORD BY SECURE COMPUTATION PRODUCT-SUM ～S312

CALCULATE DEATH RISK THAT IS EXPONENTIAL VALUE OF PRODUCT-SUM VALUE BY SECURE COMPUTATION MAPPING ～S313

CALCULATE SUM OF DEATH RISKS FOR j WITH RECORD NUMBER $i <= j$ USING SECURE COMPUTATION SUM FOR $i = 1, \cdots, N$ ～S314

CALCULATE LOGARITHMIC VALUE OF SUM OF DEATH RISKS BY SECURE COMPUTATION MAPPING ～S315

CALCULATE DIFFERENCE BETWEEN PRODUCT-SUM VALUE AND LOGARITHMIC VALUE BY SUBTRACTION OF SECURE COMPUTATION ～S316

CALCULATE LOG PARTIAL LIKELIHOOD FROM SURVIVAL OR DEATH INFORMATION AND RESULT OF SUBTRACTION USING SUM OF PRODUCTS OF SECURE COMPUTATION ～S317

END

# FIG.9

EP 4 708 261 A1

## INTERNATIONAL SEARCH REPORT

| | International application No. |
|---|---|
| | **PCT/JP2024/008140** |

### A. CLASSIFICATION OF SUBJECT MATTER

*G09C 1/00*(2006.01)i
FI:   G09C1/00 650Z

According to International Patent Classification (IPC) or to both national classification and IPC

### B. FIELDS SEARCHED

Minimum documentation searched (classification system followed by classification symbols)

G09C1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2022/079904 A1 (NIPPON TELEGRAPH AND TELEPHONE CORPORATION) 21 April 2022 (2022-04-21) | 1-6 |
| A | CN 111506922 A (ALIPAY (HANGZHOU) TECHNOLOGY CO., LTD.) 07 August 2020 (2020-08-07) | 1-6 |
| A | 川崎 将平 他, プライバシを保護した尤度比検定, 2014年度 人工知能学会全国大会（第28回）論文集, May 2014, 2B5-OS-15b-3, pp. 1-4, (KAWASAKI, Shohei et al. Privacy-preserving likelihood-ratio test.), non-official translation (Proceedings of 28th Annual Conference of the Japanese Society for Artificial Intelligence (2014)) | 1-6 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **30 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/008140**

| Patent document cited in search report | Publication date (day/month/year) | Patent family member(s) | Publication date (day/month/year) |
|---|---|---|---|
| WO 2022/079904 A1 | 21 April 2022 | US 2023/0367846 A1 | |
| | | EP 4231272 A1 | |
| | | CN 116324935 A | |
| CN 111506922 A | 07 August 2020 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019124260 A
- JP 2020042128 A
- JP 2008299370 A

**Non-patent literature cited in the description**

- System of Secure Computation and Principles thereof. *NTT Corp.*, 06 January 2023, https://www.rd. ntt/sil/project/sc/secure_computation.h tml>